# EUROPEAN PATENT APPLICATION

(11) **EP 0 583 151 A1**
(43) Date of publication of application: **16.02.1994**
(21) Application number: 93306302.6
(22) Date of filing: 10.08.1993
(51) Int. Cl.: A61B 17/12

(54) **Hemostatic clip cartridge with clip retaining means**

(30) Priority: 12.08.1992 US 929632
(71) Applicant: EDWARD WECK INC., Research Triangle Park, North Carolina 27709 (US)
(72) Inventor: Taylor, Warren, Cary, North Carolina 27511 (US)
(74) Representative: Thomas, Roger Tamlyn

(57) **Abstract**

A holder for storing and retaining a plurality of hemostatic clips. The holder (100) is produced as an integrally molded unitary piece having a plurality of longitudinally spaced chambers (118), each having a central post to support a clip and integrally formed guiding means for guiding the jaws of an associated forceps-type clip applier by which a clip within a selected chamber may be removed from the cartridge. A resilient projection (181) is formed in the transverse walls of each clip chamber on each side of the center post at a point spaced a predetermined distance above each clip leg of an open clip retained in the chamber.

## Description

The invention relates generally to holders for storing and retaining hemostatic clips prior to use. More particularly, the invention relates to hemostatic clip cartridges which facilitate the storage and retention of a plurality of hemostatic clips within a corresponding plurality of individual chambers of the cartridge prior to withdrawal of the clips by a forceps-type clip applier.

Hemostatic clips have long been used to ligate or clamp blood vessels during surgical procedures. The clips are generally made of either a biocompatible metallic material or of a polymeric or non-metallic material. The term "non-metallic" as used herein means anything other than metallic materials. In general, the clips are generally C-shaped, U-shaped or V-shaped and are designed such that the opposing legs of the open clip are able to be closed together by being compressed by the jaws of a forceps-type clip applier. In the case of metallic clips, the material is sufficiently strong such that the clips remain in their crimped, closed position merely by squeezing the legs together. In the case of plastic-type clips, the material is not as strong and requires some auxiliary latching mechanism to keep the clips closed. For the foregoing reasons, metallic clips are generally symmetrical while plastic clips are not. The latching mechanism generally comprises a hook-type arrangement molded with or otherwise secured to the plastic clip. One example of such a plastic ligating clip is shown in U.S. Patent 4,834,096 (Oh et al.) assigned to the assignee hereof.

Because the clips are small and because many clips are usually used in a surgical procedure, holding devices are used to store and retain the clips -- whether metallic or plastic -- between the time of their manufacture and ultimate use in a surgical procedure. While numerous clip cartridges are known, they all serve to prevent the clips from becoming loosened during shipment and handling and from becoming completely dislodged. A distinction should be made between clip cartridges intended for use with "manual" clip appliers and those intended for use with "automatic" clip appliers. As used herein, the term "automatic" means those clip appliers which retain a plurality of hemostatic clips adjacent the jaws of a clip applier in a way such that a new clip is automatically fed to the jaws after the previous clip has been crimped into place. As used herein, the term "manual" means clip appliers which receive one clip at a time between the jaws and which have to be reloaded manually after the previous clip is crimped. The reloading operation is generally accomplished by inserting the jaws of the applier into a clip holder or cartridge which is generally provided with a plurality of longitudinally spaced, clip retaining chambers. A single clip is retained in each chamber by a variety of means and removed from its chamber by a forceps-type clip applier which is inserted as desired into each clip chamber and secured to the clip sufficiently to overcome whatever clip retention means is utilized to enable the clip to be removed from the clip chamber.

Various mechanisms are known by which clips may be retained within the chambers of clip cartridges. With respect to metallic clips, friction between the clip and the side walls of its individual chamber is generally sufficient to retain the clip. The clip cartridges are generally made of molded plastic material such that the walls of each clip chamber are somewhat resilient and able to be pushed away from each other when the clip applier jaws are inserted into the chamber to retrieve the clip. An example of a cartridge holding the clips in their respective clip chambers by means of frictional engagement with the side walls of each chamber is shown in U.S. Patent 4,076,120 (Carroll et al.). In some prior art clip cartridges, each individual clip chamber is provided with a central post generally conforming to the shape of the open clip although being slightly larger so that when the clip is pushed onto the central post, frictional contact between the legs of the clip and the central post retains the clip within its chamber. Cartridges of this type are shown in U.S. Patent Nos. 3,270,745, 3,326,216, 3,363,628, 3,439,522 and 3,439,523, all issued to E.C. Wood.

Prior art cartridges are also known which retain clips in a partially straightened state by maintaining each clip under tension within its chamber by the interaction between the central post in the chamber and the central part of the clip and protrusions extending into each chamber toward the central post (from the ends). The clip is retained by having its central hinge part pushed upwardly by the central post and its ends pushed downwardly by the protrusions. Such a cartridge is shown in U.S. Patent 3,713,533 (Reimels) and U.S. Patent 4,146,130 (Samuels et al.)

Another type of prior art cartridge is known which has a plurality of ribs extending from each side wall of each clip chamber inwardly toward the clip to retain the clip by frictional engagement with the ribs (U.S. Patent 4,696,396, Samuels). The aforementioned U.S. Patent 4,146,130 (Samuels et al.) shows an alternative embodiment for the situation where clips are intended to be loosely maintained in the cartridge without frictional engagement between it and the chamber, the clips in such an event being retained in each cartridge by a covering tape which may be easily severed by the applier as desired.

With respect to non-metallic clips, the prior art cartridges suitable for holding metallic clips are not necessarily suitable because the plastic clips inherently have a greater resiliency and non-symmetrical structure. For example, the aforementioned prior art cartridges which rely on the interaction between the clip and central post are not suitable for use with non-metallic clips because there is generally insufficient compression in non-metallic clips to cause them to grip the center post adequately. Likewise, frictional engagement with either the side and/or end walls of the chamber could possibly, over a long period of storage time, adversely affect performance of the clips. It would be preferable to retain non-metallic clips in a natural, relaxed state without any external stress applied to the clips until they are ready for use.

One known prior art method of holding plastic clips is shown in U.S. Patent 4,294,355 (Jewusiak et al.) which discloses one or more resilient fingers associated with each clip chamber for holding each,clip in a particular fixed orientation. The entire cartridge may or may not be covered by a thin film having a plurality of lines of weakness over each clip to identify the clip location for easy retrieval by the clip applier.

U.S. Patent 4,361,229 (Mericle) discloses a cartridge for non-metallic clips wherein each clip is retained in its individual chamber by the interaction between a central post supporting each clip and paper flaps extending into each end of each chamber, the paper flaps being formed from a paper film interposed between a base portion of the cartridge (to which the central post is secured) and a top portion.

U.S. Patent 4,961,499 (Kulp) discloses a clip cartridge in which each clip is retained in its individual chamber by frictional engagement of the hinge portion of each clip between two inwardly extending ribs situated at the top of each center post.

Our European Patent Application EP-A-482861 discloses a clip cartridge in which the clips are retained in their individual chambers without frictional engagement because of inwardly extending ribs which are spaced above the center post.

The known prior art cartridges suitable for non-metallic clips are relatively complex to manufacture and load and there is a need for a hemostatic clip holder of simplified structure which also simplifies the loading of clips into the holder.

The preferred forms of the invention hereinafter described in detail provide:-
(a) a hemostatic clip holder for retaining clips during shipping and handling while enabling the withdrawal of the clips by clip appliers in preparation for use;
(b) a unitary clip holder for storing and retaining a plurality of hemostatic clips for subsequent removal by an associated clip applier;
(c) a hemostatic clip holder for storing and retaining a non-metallic hemostatic clip for subsequent removal by an associated clip applier;
(d) a hemostatic clip cartridge for storing and retaining a plurality of non-metallic hemostatic clips in a natural, relaxed state prior to their subsequent removal by an associated clip applier.

According to one aspect this invention provides a cartridge for holding hemostatic clips comprising an elongated body forming a plurality of longitudinally spaced chambers, each chamber for receiving a hemostatic clip. Each chamber has a central post for supporting an open hemostatic clip and a pair of facing transverse walls extending across the body. Preferably, a single resilient tab is formed in each transverse wall, each tab having a projection that extends into the chamber from each side of the central post at a point spaced a predetermined distance above each leg of an open clip retained in the chamber.

Preferred embodiments of the invention will now be described with reference to the accompanying drawings in which:-

Figure 1 is a perspective view of a hemostatic clip cartridge as disclosed in EP-A-482861.

Figure 2 is a side elevational view of the cartridge of Figure 1.

Figure 3 is a side elevational view of a hemostatic clip cartridge similar to that shown in Figure 2, but incorporating the invention disclosed herein.

Figure 4 is a top plan view of Figure 3.

Figure 5 is a cross-sectional view of Figure 3 taken along the lines 5-5 with the addition of a clip in place on the center post.

Figure 6 is a front perspective view of Figure 5 without the clip.

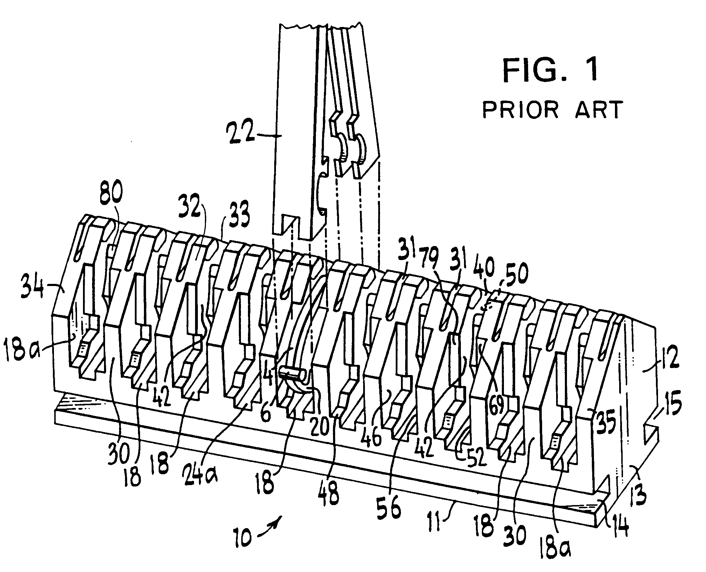

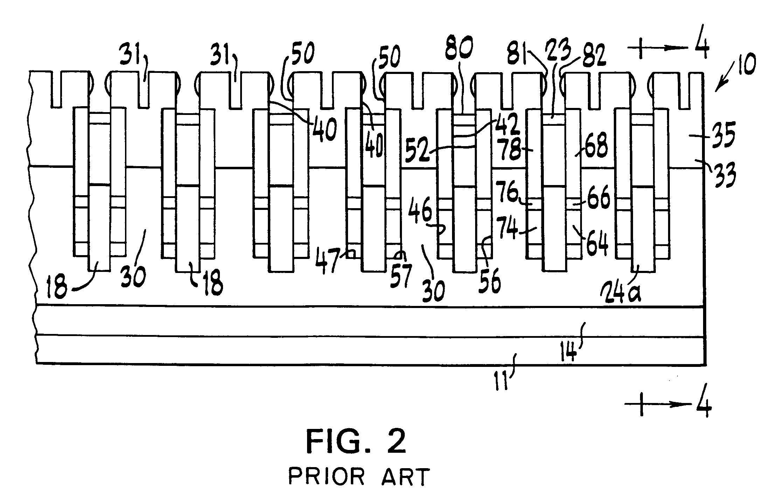

Referring now to Figures 1 and 2, there is shown a prior art clip cartridge as disclosed in the aforementioned co-pending patent application. This cartridge is shown to clarify the relationship of various component parts to be described below with respect to this invention.

Prior art clip cartridge 10 is a single molded plastic component comprising a plurality of longitudinally spaced, individual clip retaining chambers 18, each of which is identical and retains a clip 20 (only one of which is shown). Clip 20 is a non-symmetrical hemostatic clip of the type shown in U.S. Patent 4,834,096 (Oh et al.), assigned to the assignee hereof and incorporated by reference herein. As best seen in Figure 1 and in the aforementioned '096 patent, clip 20 comprises an integral plastic C-shaped body 4 with two legs hinged at a central point and a pair of lateral engagement means or bosses 6 near the ends of each leg. As will be understood below, bosses 6 interact with portions of cartridge 10.

Cartridge 10 has a base portion 11, a body portion 12 and a neck portion 13 interposed between the body and base in order to define channels 14 and 15 which serve to facilitate securing the cartridge to a tray or other component during use. It will be noted that cartridge 10 is symmetrical about its longitudinal centerline and this facilitates its manufacture as well as its assembly with clips 20. The jaw portion of a representative clip applier 22 is diagrammatically shown in position over clip 20 in one of the clip chambers 18.

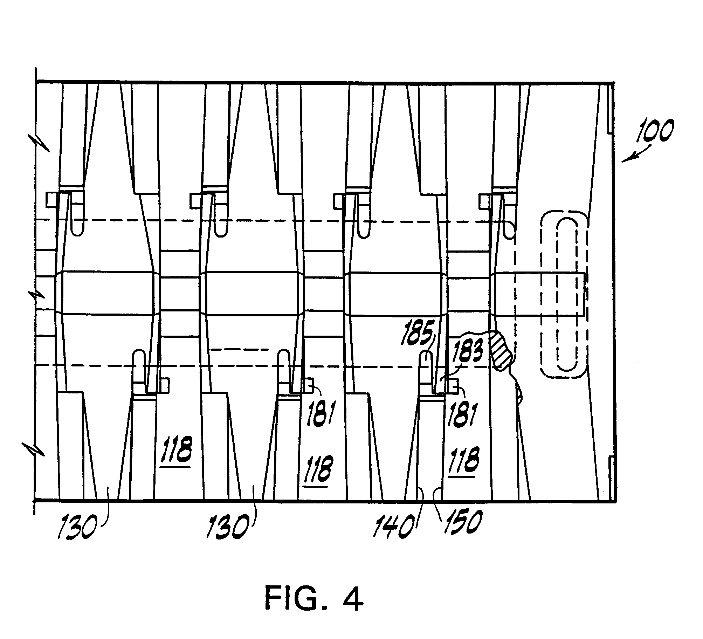

Adjacent clip chambers 18 are separated by transverse walls 30 which (at their bases) extend across the width of body 12 (best seen in Figure 4). The end clip chambers 18a obviously have a wall 30 only on one side and are bounded on the opposite side by end wall portions 34 and 35 of body 12, as the case may be. These end wall portions have some similarity to walls 30 in order to insure that end chambers 18a are identical in operation to intermediate chamber 18. Each wall 30 and end walls 34 and 35 have a transverse slit 31 near their upper ends, the purpose of which will be discussed below. The upper surface of each wall 30 is tapered at 32 and 33 to reduce cartridge mass and improve the accessibility of the clips.

Each wall 30 has a pair of symmetrical and oppositely facing transverse walls 40 and 50 which form the sides of each chamber 18: surface 40 facing inwardly into one clip chamber 18 and surface 50 facing inwardly into an adjacent clip chamber 18. For simplicity, the walls facing to the right in Figure 2 are each identified as 40 and those facing to the left are each identified as 50. Each clip chamber 18 is also provided with an integral central post 23, interposed between facing walls 40 and 50, and has floor portions 24a and 24b on either side of the post. Walls 40 and 50 are formed by a variety of facing symmetrical and parallel wall and ledge surfaces.

It will be understood that the features of wall 50 have symmetrical counterparts on wall 40. Wall 50 includes a main portion 52 which is spaced from its symmetrical facing surface 42 by an amount slightly greater than the width of the body 4 of clip 20 to be received in chamber 18. Wall 50 has recessed wall portions 56 symmetrically arranged on either side of main portion 52, and wall 40 has facing recessed wall portions 46. The distance between facing surfaces 46 and 56 is greater than the spacing between facing main portions 42 and 52. The difference in these spacings accommodates lateral bosses 6, creating longitudinally extending ledges perpendicular to wall portions 46, and 56, the purpose of which will be discussed below, and provides access to the jaws of clip applier 22 while limiting any twisting of clips 20 in their chambers.

Spaced above the upper tip 80 of central post 23 are inwardly directed, facing and symmetrical protrusions 81 and 82 integrally formed near the apices of surfaces 40 and 50, respectively. Protrusions 81 and 82 serve as clip retaining means by slightly narrowing the distance between faces 40 and 50 in part of the area above central post 23.

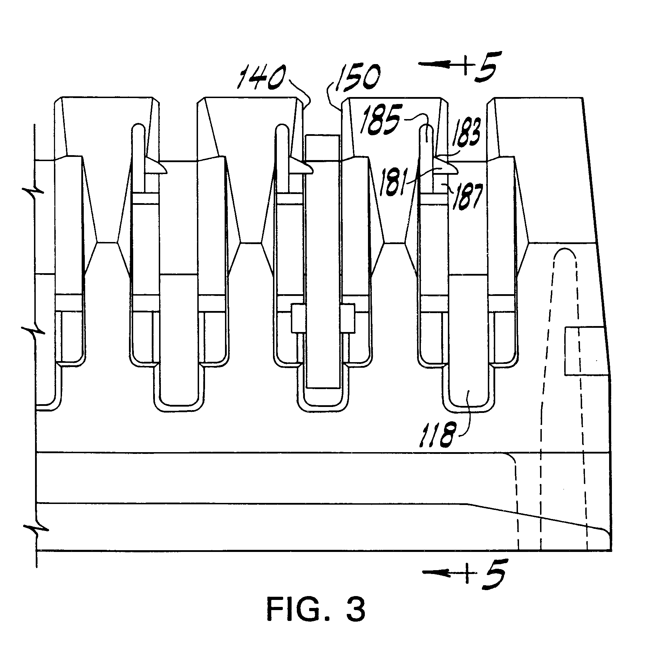

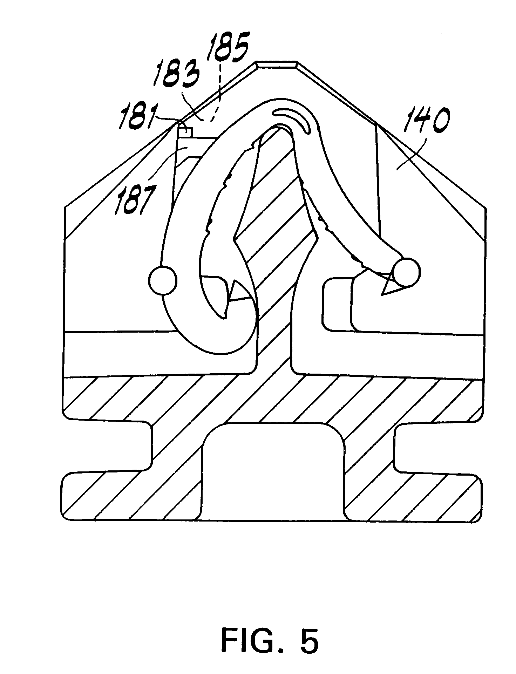

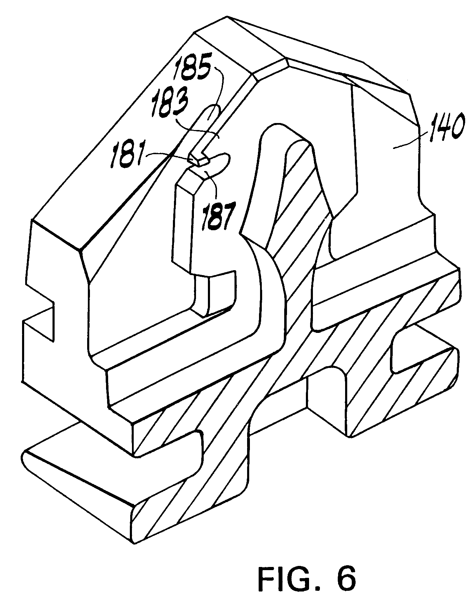

While the foregoing description describes a prior art device, the present invention is described below and shown in Figures 3 through 6 with respect to clip cartridge 100. Since clip cartridge 100 is in many respects identical to clip cartridge 10, only those features of cartridge 100 which differ significantly from similar features of cartridge 10 will be described. Other, non-described features of cartridge 100 may be understood by reference to corresponding parts of cartridge 10.

The primary difference between cartridge 10 and the new cartridge 100 lies in the replacement of axially disposed protrusions 81 and 82 by a single off-centered projection 181 extending inwardly into each clip chamber 118. Thus, the wall 140 is similar to wall 40 except for the difference in this clip retaining feature. Projection 181 is situated at the end of a resilient arm 183 integrally molded into cartridge 100. While the material from which the preferred embodiment of cartridge 100 is molded is relatively hard (i.e. polycarbonate), arm 183 is somewhat resilient by virtue of cutouts 185 and 187 which result in the arm being attached to the cartridge only along its proximal end. The distal tip of arm 183 is provided with integrally molded projection 181 which extends into clip chamber 118 a sufficient distance in order to project over any clip that may be present in chamber 118. Two projections 181 are situated in each chamber, one in each side of the center post. It is equally feasible for there to be more than one projection on each side of the center post, depending on the size or shape of the clip intended to be used in the cartridge.

## Claims

1. A cartridge for holding hemostatic clips comprising:
an elongated body forming a plurality of longitudinally spaced chambers, each chamber for receiving a hemostatic clip and comprising:
a central post for supporting an open hemostatic clip, and
a pair of facing transverse walls extending across said body, each of said transverse walls having at least one resilient projection means for extending a predetermined distance into said chamber above a clip situated therein and on a predetermined side of said post.

2. A cartridge according to claim 1 wherein said resilient projection means is situated a predetermined lateral distance from said post in order to be situated above a predetermined portion of a clip retained in said chamber.

3. A cartridge according to claim 1 or 2 wherein each of said transverse walls further comprise an inwardly extending resilient projection means forming a pair of oppositely facing projections extending into the associated chamber at a predetermined distance below the top of said center post, one on each side thereof, and arranged to be deflected by a clip as it is removed from said chamber.

4. A clip cartridge in accordance with claim 1, 2 or 3 wherein said at least one resilient projection means comprises:
an arm portion integrally molded with said transverse wall , said arm portion having a distal end and a proximal end, said proximal end being attached to said cartridge;
a protrusion at said distal end of said arm portion, said protrusion extending into said chamber a predetermined distance sufficient to prevent a clip situated therein from being removed from said chamber without deflecting said arm portion.

5. A clip cartridge according to any preceding claim wherein said resilient projection means is situated below the top of said center post and above the leg of a clip situated in said chamber.
